Europäisches Patentamt

European Patent Office

Office européen des brevets

(19)

(11) Publication number:
**0 322 392**
**A1**

(12)

# EUROPEAN PATENT APPLICATION

(21) Application number: **88850432.1**

(22) Date of filing: **16.12.88**

(51) Int. Cl.⁴: **A 61 K 9/00**
**A 61 K 9/22, A 61 K 9/26**

(30) Priority: **22.12.87 SE 8705136**

(43) Date of publication of application:
**28.06.89 Bulletin 89/26**

(84) Designated Contracting States:
**AT BE CH DE ES FR GB GR IT LI LU NL SE**

(71) Applicant: **PHARMACIA AB**
**Rapsgatan 7**
**S-751 82 Uppsala (SE)**

(72) Inventor: **Kristensen, Arne**
**Askvägen 22 A**
**S-752 52 Uppsala (SE)**

(74) Representative: **Engholm, Carl et al**
**Pharmacia AB Patent Department Box 604**
**S-751 25 Uppsala (SE)**

(54) Oral dosage units for pharmaceuticals and their use and preparation.

(57) The invention relates to an oral dosage unit of a pharmaceutical formed as small particles (< 2,5 mm in diameter), in which the particles are partitioned in a gel with pH < 5, which exist as a shaped body and which is disintegrable in the gastro intestinal tract.
  In addition, the preparation and use of the dosage unit are described.

EP 0 322 392 A1

**Description**

## Oral dosage units for pharmaceuticals and their use and preparation

The invention relates to a preparation form for oral administration of separate dosage units of a pharmaceutical. The invention is primarily meant for human use, but can also be used to administrate pharmaceuticals to animals.

Tablets and capsules are the most important preparation forms for oral administration of separate dosage units of a pharmaceutical. The choice of preparation form depends on demands of dosage precision, type of active substance, where in the body the activity should take place, the form of the active substance etc. In oral administration, factors such as taste, smell, ability to swallow and absorption in mouth, stomach, doudenum and small or large intestine are of great importance. For release in the intestine, it is well known to use gastric juice resistant preparation forms and other preparation forms with prolonged, but well defined, release. For a review on the subject, see Sustained Release Medications, ed. Johnson J.C., Noyes Data Corp., USA, 1980. When pharmaceutical substances and the desired effect allows it, preparation forms such as gel, oil, solution, emulsion etc. occur. These latter forms imply a significantly lower accuracy in dosage than tablets and capsules, since it often takes place on the level of patient. A gelatinous preparation form especially adapted for children has earlier been described (DE-A-2, 105, 110). To our knowledge, this preparation has not gained any wider practical use. Pharmaceuticals formed in moulded bodies have recently been described (DE-A-3, 545, 090). However, how to solve the stability problems, which in water containing media are obvious for gastric juice resistant preparations are not considered. In GB-A-2084871 a shaped body, consisting of a polymer - water mixture, in which particles with gel-chromatographical qualities and containing incorporated pharmaceutical active substances is described. Gastric juice resistant preparations are not described in GB-A-2084871. More or less dry shaped bodies designated for pharmaceutical administration to the eye are described in US-A-3867519.

Increased knowledge about the physiology of the gastro intestinal tract has lately resulted in new and increased demands on preparation forms of pharmaceuticals meant for oral administration. By a series of investigations it is considered today to be documented that the passage time for a gastric juice resistant compressed particle varies between 1 and 18 hours before it reaches the small intestine. This was earlier calculated to be in the order of 1 to 4 hours. For pharmaceuticals which shall be absorbed or in any other way perform their action in the intestine, this implies that large variations can arise. Absorption of pharmaceuticals administrated in a form of compressed particles, has been shown to be strongly dependent on the emptying of the ventricle. For particles larger than 1-2 mm, the velocity of absorption in the intestine has also been shown to be dependent on the patient's age and status of health, the size and composition of the meal, the time between intake of the tablet and finishing of the meal, the amount of liquid and pH value in the ventricle. On the other hand, the passage time through the small intestine is considered to be almost constant and very little dependent on the size of the passing units. Thus should solid preparation forms, that shall be absorbed in the intestine, have a particle size less than 2 mm. This has led to preparation of capsules containing a great number of small particles (pellets), each coated with an envelope.

Pharmaceuticals, formed as compressed particles, or other solid particulate forms aimed for oral intake under longer time periods, are considered to cause the patient great inconvenience. This is the case with tablets, capsules and smaller particles in the form of "sachets".

The invention presents a new oral preparation form for a dosage unit of a pharmaceutical. The invention also includes a method to manufacture the preparation form and a method to orally administrate a pharmaceutical. The preparation form of the invention shows improved qualities with regard to the above mentioned drawbacks with earlier known particulate preparation forms, which have prolonged release in the gastro intestinal tract. The preparation form of the invention has as a characteristical quality that a dosage unit of the pharmaceutical is formed like smaller particles ( < 2,5 mm in diameter) with prolonged release of active substance, which is partitioned in a gel which exists as a shaped body. The term "shaped body" means that it has a defined shape (cube, sphere, oval etc.), which it can maintain when stored free. The shaped body can decompose and/or dissolve when it is transported through the gastro intestinal tract, in which it is exposed to different physiological conditions.

The decomposition/dissolution can be mechanical and/or due to changes in pH, temperature, presence of enzymes etc., and serves the purpose to disclose the separate particles so that they easily should be transported and adsorbed in the gastro intestinal tract. Usually, as gel, the shaped body is more or less elastic. The shaped body can be a tixotroph gel, so that it upon chewing easily converts into a fluid form, but this does not have to be the case. To make the swallowing of the shaped body easy, it is essential that it can be formed or decomposed in the mouth. The gel should be stable during storage and may thus be free from syneresis. Many of the current gels often easily loose or take up water, which implies that their physiological quality and geometrical shape can change during storage if necessary actions are not taken. Therefore it can in many cases be essential that the shaped body is air- and moisture free packed. As a rule, the particles have a homogenous size distribution, are usually larger than 0,1 mm, rigid (solid) and undissolved in the shaped body. The particles can be rigid and undissolved as such or have an envelope which gives them these qualities. If the envelope is lacking, they are always of the non-gel type. The following is available for the current gels; they should show a melting temperature, i.e. the temperature when the gel is converted into a colloidal solution, which is above 37 °C, as above 50 °C or above 60 °C. A melting point at about 35 °C - 40 °C means that the gel is melting at the oral intake, which can facilitate the swallowing. Though such a low melting point implies that one must guarantee that the storage temperature does not exceed 37 °C. There are a number of different substances which can form gels of the current type. Usually these substances are hydrophilic polymers, based on in nature existing polymers (proteins and carbohydrates). They form colloidal solutions in water, which can be converted into gel by changes in temperature, pH and/or by addition of salt, sugar or other for a defined polymer known and for gel formation essential agent. Suitable polymers are mainly not cross-linked. Many of the current polymers contain sulfate-, carboxylic acid- or amin structures or other groups, whose changes vary dependent on the pH value. This entails that the formation of gel in many cases is dependent on pH and the salt added, which is especially important for certain proteins, like gelatine and certain polysacharides, e.g. agar, carrageen and pectin. The following is available for the preparation form of the invention, the pH in the shaped body (the gel) should be adjusted to a pH acceptable for the gastro intestinal tract, which means below 5 but the preferred value lies below 4,5. This can be done using citric acid -phosphate buffer. The citric acid can alternatively be replaced with other physiological preferred di- or polycarboxylic acids, such as tartaric acid, adipic acid etc. The lower limit for the pH value in the gel is determined by the polymers and other components' stbility against i.a. hydrolysis. The pH in the shaped body (gel) should as a rule be above 2,5, since too low pH could lead to degradation of the gel forming polymer. Incorrect pH can for certain polymers lead to precipitation in the gel. If the gel forming polymer is a polysacharide, it is well known that to improve the quality of the gel, mono- or disaccharides, sugar-alcohols (e.g. xylitol and glycerol), $Ca^{+2}$ etc. can be added. Especially it is worth mentioning that $k^+$ is known to increase the strength of the gel, e.g. in gels of pectin. Usually, as a counter-ion to $k^+$, the pH adjusted acid component is used, but alternatively could the counter-ion be another than $k^+$ or combination of different counter-ions.

Many of the current bio-polymers can be used in chemically modified form and covered by the invention. When pH is adjusted according to the invention, several practical advantages are gained, such as improved stability, taste and automatic secretion of salivary when the preparation is consumed. Flavouring and preservatives can be added.

For gel formation of the current polymers, water is needed. For a given gel forming system (polymer, pH, rest of addidatives), the gel's physical quality is determined by the water content. Too little water easily gives leathery gels, which are unpleasant to chew. Too much water gives loose gels, which do not give shaped bodies as above. The necessary water content varies i.a. dependent on polymer and lies as a rule below 80 %, such as below 40 %. For hydrolysis, sensitive pharmaceutical preparations it is essential that the water content is as low as possible, e.g. below 30 % and/or that pH is adjusted to a for the stability optimal pH, but at the same time consideration of the used polymers demands a pH for gel formation and stability. Since it is possible to produce gels which in principle are devoid of water, the lower limit for water content is 0 %. However, usually it is above 5 %. With our present knowledge about the invention's design, water contents at 15-30 % (especially for pectins) are preferred. The sacharose content should be kept as low as possible, preferably below 50 %. The per cent content above refers to w/w and is calculated for the final dosage unit.

Many of the current polymers are used in the food industry as consistency formers, e.g. in marmalades, jams, ice-cream, cream, assorted sweets and chocolates. The information about different polymers can be obtained from the respective manufacturers and from encyclopedias within the field (e.g. Food Hydro Colloids, CLC Press Inc. Boca Raton, Florida). Current polysacharides to be used are in English often referred to as "gum" (see e.g. Encyclopedia of Polymer Science and Engineering, vol. 7, John Wiley & Sons, 589-613).

With our present knowledge, the pectins are the most preferred polymers.

## Active pharmaceutical substances and their formulation in gel

The invention is applied on a large number of pharmaceutical substances intended for oral administration. Generally it is an ambition that the particles containing the active substance shall be partitioned as homogenously as possible in the gel. Depending on the active substance and where in the body it should be absorbed, the type of mouldability in the gel of the shaped body is chosen.

Starting with the colloidal polymer solution, the particulate and in water undissolvable pharmaceutical preparation is dispersed. The particles (microcristallic form, granulate, pellets etc.) are designed for release of active substance on intended place in the gastro intestinal tract. Thus they can be coated with a film, which is resistant (e.g. hydrolysis-stable) against juice from stomach, doudenum and/or the juice of small intestine. Special interest concerns films which are stable, but specifically could be degraded under the conditions that exist in any place in the gastro intestinal tract. The demands on gastric juice- and intestine juice resistant preparations are in detail documented in different pharmacopoeia, see e.g. USP XXI (1985), chapter 724 (Drug Release). In this matter it has lately been discussed whether hydrolysis stable films as reductively can be degraded in the large intestine. Among the examples, diazo-polymers have been mentioned.

The pharmaceutical preparation incorporated in the gel usually constitutes less than 40, 30 or 20 % (w/w). In the normal case, the incorporated pharmaceutical preparation does not exceed 35 % (w/w) of the final weight of the shaped body. Usually, when intended for human use, the total weight of the shaped body in the invention is less than 10 g, but depending on the incorporated pharmaceutical preparation and on the age of the patient to whom it should be given, less than e.g. 7 g or less than 5 g. As a lower limit 0,3 g is valid. For other animal species, other limits are valid. For large animals, like horses and cows, it is relevant with shaped bodies up to 200 g, while hamsters and small dogs (papillon) are satisfied with significantly smaller shaped bodies.

In the cases where gel forming polymers require addition of mono- or disacharide (I, II) or sugar alcohol (III), the pharmaceutical preparation usually constitutes less than 30 % (w/w) of the shaped body and has replaced corresponding amount of I, II or III.

The invention's dosage unit is prepared according to:

1. A gel forming polymer, a suitable buffer system and other addidatives, which are necessary for the formation of the gel, are mixed with water to a homogenous solution or a colloidal system. When necessary, the mixture is concentrated afterwards. Everything in this step is done in a manner well known for respective polymer. The amount of dry substance is determined (e.g. refractometrical) to control the process. Too low or all too high an amount of dry substance can lead to gels with qualities other than the intended.

2. The coated pharmaceutical preparation (microcristalline form, pellet etc.) containing the active substances is homogenously partitioned to that in step 1 obtained mixture.

3. Then the mixture is partitioned in equal-sized moulds and allowed to solidify. The obtained shaped bodies are then packed air- and moisture tight and eventually also light tight.

As a rule step 1 and 2 are done under an elevated temperature (above about 37 °C) and step 3 at or below room temperature.

In certain cases it is practical to let the mass from step 2 cool down to room temperature, before step 3 is done. For many gel forming polymers, one can add agents which slow down the solidification of the gel. Calcium phosphate is an example for certain qualities of pectin. Compounds, which via chemical reaction affect a solution of a gel forming polymer, so that conditions slowly change from non-gel forming to gel forming, is another exemple. A number of critical points, which in themselves are known, are present in the process. Concentration of the solution in step 1 is not done so that sediment formation occurs. Buffering to optimal pH must be done with carefulness. Certain polymers are sensitive to hydrolysis, and excess of acid or base can easily lead to deterioration in corresponding gels' rheological qualities. Similar difficulties can also arise if the amount of polymer is chosen too high or too low. For commercially accessable polymers, the manufacturers usually can inform about the practical pitfalls that eventually are present.

The invention also includes a way to administrate pharmaceuticals, and implies that the pharmaceutical is administrated as a dosage unit according to the invention. This aspect of the invention is primarily intended for administration to animals which belong to Mammalians as human.

Examples of active substances, that potentially can be mixed in the gel during step 2, are: Salicylazo-sulpha pyridine, Acetyl salicylic acid, 5-ASA, Diklofenac, Naproxene, Procain amide, Tetracyclines, Erythromycine, Aminosalyl, Kinidin, Hyoscyamine, Potassium, derivates of Penicillin, Morphine (pethidine), Butazolidine, Propranolol, Indomethazine, Furosemid.

The invention is defined by enclosed patent claims, which constitute a part of the description and shall now be clarified with a number of examples.

### RECIPE

1. Pectin
   (i) Buffered LM-pectin
   (ii) HM-pectin
2. Agar
3. Carrageenan
   (i) Genugel Type LC-4
   (ii) Genugel Type LC-5
4. Gelatine

Declared carrageenan- and pectin qualities have been obtained from A/S Københavns Pektinfabrik, Denmark. LM and HM designates low-ester- and high-ester pectin respectively.

Gelatine has been obtained from Extraco Geltec, Klippan, Sweden.

Agar (Agar Noble) has been obtained from Difco, USA.

Pellets (placebo) came from Hans G. Werner, Dragee Fabrik, Tornesch, W-Germany.

Pellets (Dipentum®, 5,5-azo-bis-salicylic acid, diameter about 1,5 mm), have been made at Pharmacia AB, Sweden, coated with Eudragit® (10 %; from Röhm Pharma, W-Germany) and used in example 1.

Placebopellets (diameter about 1,5 mm) have been coated with HPMCP film (HPMCP = hydroxipropylmethyl cellulosaftalat, Shin-Etsu Chemical Co., Japan) and used in all examples.

Glucose syrup was 84 % (w/v)

Sugar was sacharose.

Potassium citrate can be exchanged against sodium citrate, but then a potassium salt has to be added to give the necessary strength of the gel.

1. (i) Buffered pectin-LM

| A | Water | 300 g |
|---|---|---|
| | Buffered Genu pectin Type LM-102AS-CAB | 25 g |
| | Sugar | 75 g |
| | Sugar | 195 g |
| | Glucose syrup | 300 g |
| B | Pellets | 250 g |

A colloidal solution containing the components under A was prepared according to the manufacturer's recommendations, which in general terms are written out on page 7 (step 1). Then the solution was kept at > 90 °C while pellets were added. Thereafter the composition was partitioned in moulding forms and allowed to cool ( < 90 °C).

### 1. (ii) Pectin-HM

| A | Water | 300 g |
|---|---|---|
| | Sodium citrate | 4 g |
| | Citric acid 50 % | 7,5 ml |
| | Genu pectin Type D slow set conf 150° USA-SAG confectionary | 15 g |
| | Sugar | 50 g |
| | Sugar | 210 g |
| | Glucose syrup (84 %) | 300 g |
| | Citric acid 50 % | 4,5 ml |
| | (Taste and colour) | 2,0 g) |
| D | Pellets | 250 g |

Manufacturing according to 1. (i).

### 2. Agar

| Agar | 1,0 g |
|---|---|
| Sugar | 18,0 g |
| Citric acid solution 50 % | 0,7 ml |
| Potassium citrate | 0,25 g |
| Water | 80,0 g |

The production is mainly according to the manufacturer's recommendation, although minor modifications have been done, which is why a complete description is given.
1. Distribute agar in cold water under stirring.
2. Add sugar and potassium citrate under stirring.
3. Heat to the boiling temperature.
4. Add citric acid.

5. Add from the pharmaceutical preparation (pellets). Pellets are added (25 % of the gel). The solution must cool down to about 35 °C, but absolutely not any lower, because then it becomes too lumpy and is impossible to mould. The temperature of the solution must be kept at 35 °C so that the pellets can be homogenously distributed in the solution. At higher temperatures, the pellets sink to the bottom as the solution is not viscous enough.
6. Partition the composition in the moulding forms and let it solidify.

### 3. (i) Carrageenan - Genugel Type LC-4

| Genugel type LC-4 | 1,0 g |
|---|---|
| Sugar | 16,0 g |
| Citric acid solution 50 % | 0,40 ml |
| Potassium citrate | 0,3 g |
| Water | 70,0 g |

A colloidal solution containing these components, the pellets excluded, was produced according to the manufacturer's recommendations. The method is in accordance with the general method as described on page 7 (step 1). Then the pellets were added (25 % w/w counted on the gel) and homogenously distributed. The moulding was at 50 °C.

### 3. (ii) Genugel Type LC-5

| Genugel Carrageenan type LC-5 | 1,3 g |
|---|---|
| Sugar | 18,0 g |
| Citric acid solution 50 % | 0,7 g |
| Potassium citrate | 0,25 g |
| Water | 72,0 g |
| Pellets | |

Production, see example 3 (i).

### 4. Gelatine

| Water | 138 g |
|---|---|
| Sugar | 20 g |
| Citric acid 50 % | 1,0 g |
| Gelatine P6-832-6 | 8 g |

The method has mainly been developed by us, therefore a detailed description is presented.
1. The gelatine is allowed to swell in cold water for one hour (8 g gelatine in 26 g cold water).
2. 20 g sugar is added to the remaining amount of water. Heat to the boiling temperature, but first check that the sugar i dissolved. Add the citric acid.

3. A little of the hot-water mixture is added to the swelled gelatine. Stir and let the gelatine dissolve a little.

4. Add the mixture of step 3 to the remaining hot-water mixture.

5. Stir until all the gelatine is dissolved.

6. The solution is partitioned in two equal parts, I and II respectively.

I. At first the solution is kept unstirred for a while (45-60 min) so that it becomes viscous enough to allow the pellets to get evenly distributed and not sink to the bottom. Thereafter the pellets (25 % of Solution I) are added.

II 225 $\mu$l NaOH 10 % to 25 g solution and then it is kept unstirred (45-60 min) so that it becomes viscous enough to allow the pellets to get evenly distributed and not sink to the bottom. Thereafter the pellets (25 % counted on Solution II) are added.

$pH_I$ = 3,59, $pH_{II}$ = 4,63.

7. The solutions are partitioned into moulding forms and allowed to stiffen.

There seems to be no difference between the two gelatine gels concerning degree of hardness and time to stiffen. Both take about 1-2 hours. Although, with a somewhat less amount of gelatine (5 g) gels with lower pH are softer in consistency than those with higher pH.

Example 4

Compositions in accordance with example 1-3 were later given orally to six different persons. They found that pectin gels were from consistency point of view the most preferable. Compositions could be swallowed without any intake of liquid.

The invention is defined in enclosed patent claims, which constitute a part of the description.

**Claims**

1. Oral dosage unit of a pharmaceutical, formed as small particles (< 2,5 mm in diameter), **characterized** in that the particles are partitioned in a gel with pH < 5, which exists as a shaped body and is disintegrable in the gastro intestinal tract.

2. Dosage unit according to claim 1, **characterized** in that the gel as gel-forming substance has a hydrophilic polymer which can form a water-containing gel.

3. Dosage unit according to any of the claims 1-2, **characterized** in that the polymer is a polysacharide such as agar, carrageenan, pectin etc.

4. Dosage unit according to any of the claims 1-2, **characterized** in that the polymer is a protein such as gelatine.

5. Dosage unit according to any of the claims 1-4, **characterized** in that the pharmaceutical is formed as many small particles with defined release of the pharmaceutical in the stomach, doudenum, small intestine or large intestine.

6. Method for production of a dosage unit of an oral preparation form containing a pharmaceutical, **characterized** in that the pharmaceutical in the form of coated small particles (diameter < 2,5 mm) is dispersed into a solution with ability to form a "selfcarrying" gel with pH < 5, whereafter the solution is partitioned as dosage units in equal-sized moulds and is allowed to form the gel.

7. Method according to claim 6, **characterized** in that the particles have a defined release of the pharmaceutical in stomach, doudenum, small intestine or large intestine.

8. The use of a preparation of a pharmaceutical according to any of the claims 1-6, for oral administration of pharmaceuticals.

European Patent Office

**PARTIAL EUROPEAN SEARCH REPORT**

which under Rule 45 of the European Patent Convention
shall be considered, for the purposes of subsequent
proceedings, as the European search report

Application number

EP88850432.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-21 05 110 (CRT CENTRE DE RECHERCHE THERAPEUTIQE) <br> * pages 2-3; example * <br> --- | 1-7 | A 61 K 9/00 <br><br> A 61 K 9/22 <br><br> A 61 K 9/26 |
| X | US-A-4 434 153 (J URQUHART ET AL) <br> * column 4, line 10 - column 5, line 5; column 7 * <br> --- | 1-7 | |
| X | EP-A-0 080 330 (STANDARD TELEPHONES AND CABLES LTD CO) <br> * page 3, lines 28-35; page 4, lines 5-13; page 6, lines 25-33 * <br> --- | 1,2,3, 5-7 | |
| Y | US-A-4 371 516 (G.K.E. GREGORY ET AL) <br> * see claims * <br> --- | 1-7 | |
| X | US-A-4 230 687 (L SAIR ET AL) <br> * see column 2, lines 1-20; claims * <br> --- <br><br> ./. | 1-7 | **TECHNICAL FIELDS SEARCHED (Int. Cl.4)** <br><br> A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7

Claims searched incompletely:

Claims not searched: 8

Reason for the limitation of the search: Method for treatment of the human body by therapy (see art 52(4) of the European Patent Convention).

| Place of search | Date of completion of the search | Examiner |
|---|---|---|
| STOCKHOLM | 14-03-1989 | FORSLUND N. |

European Patent Office

**PARTIAL EUROPEAN SEARCH REPORT**

Application number

EP88850432.1

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| X | US-A-4 540 602 (S MOTOMAYA ET AL)<br>* see claims *<br>--- | 1-7 | |
| Y | WO-A-87/06130 (LABORATORIES DE LA GRANGE)<br>* see page 2 *<br>--- | 1,6 | |

TECHNICAL FIELDS SEARCHED (Int. Cl.4)

EPO Form 1505.3   08.78